# FASCICULE DE BREVET EUROPEEN

(11) **EP 0 913 377 B1**
(45) Date de publication et mention de la délivrance du brevet: **05.03.2003**
(21) Numéro de dépôt: 98402494.3
(22) Date de dépôt: 07.10.1998
(51) Int. Cl.: C07C 5/27

(54) **Procédé d'isomérisation des composés aromatiques a huit atomes de carbone comprenant un recyclage**
Verfahren zur Isomerisierung von C8-Aromaten unter Verwendung einer Recyclierung
Isomerisation process for C8-aromatics including a recycle

(30) Priorité: 14.10.1997 FR 9713009; 24.11.1997 FR 9714820
(43) Date de publication de la demande: 06.05.1999
(73) Titulaire: INSTITUT FRANCAIS DU PETROLE, 92500 Rueil Malmaison (FR)
(72) Inventeur: Alario, Fabio, 92200 Neuilly sur Seine (FR); Joly, Jean-François, 69006 Lyon (FR); Magne-Drisch, Julia, 38200 Vilette de Vienne (FR); Miquel, Gérard, Allée Fleury, 69230 Saint Genis Laval (FR); Reymond, Marc, 69330 Meyzieux (FR); Coupard, Vincent, 69003 Lyon (FR)

(56) Documents cités:
- EP-A- 0 249 914
- US-A- 3 553 276
- US-A- 4 139 571
- US-A- 4 255 606
- US-A- 4 700 012

## Description

Ce domaine a déjà fait l'objet de recherches qui sont décrites notamment dans les brevets US 3538173, US 3553276, US 4128591 et US 4255606. Selon les procédés connus d'isomérisation des composés aromatiques à huit atomes de carbone, une charge pauvre en paraxylène par rapport à l'équilibre thermodynamique du mélange et riche en éthylbenzène (par rapport à ce même mélange à l'équilibre thermodynamique) est introduite dans un réacteur contenant au moins un catalyseur. Cette charge entre dans ce réacteur où des conditions de température et de pression adéquates sont maintenues pour obtenir une composition, à la sortie dudit réacteur, en composés aromatiques à huit atomes de carbone la plus proche possible de la composition dudit mélange à l'équilibre thermodynamique. D'autres documents décrivent des procédés d'isomérisation des composés aromatiques à huit atomes de carbone dans lesquels la charge à traiter est riche en xylènes et pauvre en éthylbenzène. Par "pauvre en paraxylène" et " riche en éthylbenzène " au sens de la présente description, on entend que la teneur en paraxylène est nettement inférieure et que la teneur en éthylbenzène est nettement supérieure à celle du mélange à l'équilibre thermodynamique, dans les conditions de température et de pressions considérées (le mélange étant constitué par le métaxylène, l'orthoxylène, le paraxylène et l'éthylbenzène).

De ce mélange, on sépare ensuite le para-xylène, et souvent aussi l'ortho-xylène, qui sont les isomères recherchés car ils présentent un intérêt important notamment pour l'industrie des fibres synthétiques. Le métaxylène et éventuellement l'éthylbenzène peuvent alors être recyclés vers l'entrée du réacteur d'isomérisation de façon à accroître la production de paraxylène et d'orthoxylène.

La réaction d'isomérisation des composés aromatiques à huit atomes de carbone par molécule pose cependant plusieurs problèmes engendrés par les réactions secondaires. Ainsi, outre la réaction principale d'isomérisation, on observe des réactions d'hydrogénation : hydrogénation des composés aromatiques en naphtènes. Des réactions d'ouverture des cycles naphténiques qui conduisent à la formation de paraffines ayant le même nombre d'atomes de carbone par molécule que les naphtènes dont ils sont issus. Ces paraffines peuvent subir des réactions de craquage qui conduisent à la formation de paraffines légères ayant typiquement de 3 à 5 atomes de carbone par molécule. Les composés aromatiques subissent des réactions de dismutation et de transalkylation conduisant, dans le cas des composés aromatiques à 8 atomes de carbone par molécule, à la production de benzène, de toluène, de composés aromatiques à 9 atomes de carbone par molécule (triméthylbenzènes par exemple) et de composés aromatiques plus lourds.

L'ensemble de ces réactions secondaires pénalisent fortement les rendements en produits désirés.

La quantité de produits secondaires formés (naphtènes, paraffines, benzène, toluène, composés aromatiques à 9 et 10 atomes de carbone par molécule pour l'essentiel) dépend de la nature du catalyseur et des conditions opératoires du réacteur d'isomérisation (température, pressions partielles d'hydrogène et d'hydrocarbures).

L'examen des documents antérieurs montre qu'il a été envisagé de réaliser des recyclages de certains constituants contenus dans l'effluent du réacteur d'isomérisation vers l'entrée dudit réacteur de façon à minimiser la production de produits secondaires.

Par exemple, les brevets US 3553276, US 3558173 et US 4255606 préconisent d'ajouter certains produits à la charge à traiter, afin de diminuer les pertes en produits secondaires.

Le brevet US 3553276 décrit un dispositif dans lequel on recycle du toluène de façon telle que la concentration en toluène soit maintenue au double de la concentration que l'on obtiendrait sans ce recyclage.

Le brevet US 3558173 décrit un recyclage vers l'entrée du réacteur des naphtènes à huit atomes de carbone produits par hydrogénation des composés aromatiques correspondant.

Dans la description du brevet US 4255606, de 1 à 10% en poids, par rapport à la charge totale, d'un hydrocarbure aliphatique contenant au moins 5 atomes de carbone par molécule est introduit dans la zone de réaction avec ou sans addition de toluène. Cette addition peut être assurée par un recyclage. L'hydrocarbure introduit peut aussi être un précurseur du n-pentane.

Le brevet US 4,139,571 décrit le recyclage de deux fractions hydrocarbonées : l'une dépourvue de composés aromatiques à huit atomes de carbone contient du toluène, éventuellement des naphtènes à huit atomes de carbone et également des composés plus légers tels que les paraffines ayant de 1 à 7 atomes de carbone ou les C5, C6, C7-naphtènes; l'autre fraction contient les aromatiques à huit atomes de carbone après séparation de l'isomère cible du xylène recherché.

Le procédé d'isomérisation des composés aromatiques à huit atomes de carbone par molécule selon l'invention comprend l'introduction dans la zone de réaction, avec la charge contenant les composés aromatiques à isomériser et l'hydrogène nécessaire à la réaction, d'un mélange contenant des composés de point d'ébullition d'environ 80 à 135° C comprenant au moins les composés suivants :
- au moins une paraffine à huit atomes de carbone par molécule
- au moins du benzène
- au moins du toluène
- au moins un naphtène à huit atomes de carbone par molécule.
et dépourvu d'hydrocarbures aromatiques à au moins 8 atomes de carbone et d'hydrocarbures ayant 1 à 7 atomes de carbone, ledit mélange étant obtenu par recyclage de la fraction contenant lesdits composés isolée à partir de l'effluent de sortie du réacteur d'isomérisation, en quantités telles que les pourcentages pondéraux en composés ajoutés par rapport à la charge totale qui entre dans le réacteur sont les suivants :
- le pourcentage en poids de paraffines à huit atomes de carbone par molécule est d'environ 0,1 à 10%, de préférence d'environ 0,2 à 2%,
- le pourcentage en poids de naphtènes à huit atomes de carbone par molécule est d'environ 0,5 à 15%, et de préférence d'environ 2 à 8%,
- le pourcentage en poids de toluène est d'environ 0,1 à 10%, de préférence d'environ 0,2 à 5%,
- le pourcentage en poids de benzène est d'environ 0,1 à 10%, de préférence d'environ 0,2 à 2%.

Le pourcentage en poids de composés totaux desdits composés ajoutés représente environ 0,8 à 20 % et souvent 2 à 15 % par rapport à la charge totale qui entre dans le réacteur.

De façon surprenante, l'ajout de ce mélange dans la charge à traiter permet de diminuer la production de ces mêmes produits. Notamment, cet ajout permet de diminuer la production des paraffines à huit atomes de carbone par molécule, des composés aromatiques contenant au moins 9 atomes de carbone par molécule et de diminuer voire de supprimer la production des naphtènes à huit atomes de carbone par molécule.

D'autre part, le procédé selon la présente invention permet d'utiliser le catalyseur dans des conditions de pression et de température plus basses et à des PPH (poids de charge/ poids de catalyseur/ heure) plus élevées, ce qui présente un avantage d'utilisation par rapport aux conditions d'utilisation de l'art antérieur.

Le mélange contenant des paraffines à huit atomes de carbone par molécule, du benzène, du toluène et des naphtènes à huit atomes de carbone par molécule peut être obtenu par un recyclage des paraffines à huit atomes de carbone par molécule, du benzène, du toluène et des naphtènes à huit atomes de carbone par molécule produits par la réaction. Ce mélange peut aussi être obtenu par des ajouts spécifiques de produits frais ou encore par la combinaison d'un recyclage et d'ajouts de produits frais.

Par rapport à l'art antérieur, le procédé selon l'invention présente de nombreux avantages parmi lesquels on peut citer une diminution des pertes en composés aromatiques à huit atomes de carbone par molécule par réactions secondaires parasites de dismutation, de transalkylation, d'hydrogénation et de craquage.

Dans les procédés classiques d'isomérisation des composés aromatiques à huit atomes de carbone, un mélange de xylènes et éventuellement d'éthylbenzène est mis en contact avec un catalyseur approprié, contenant généralement un métal noble et un support minéral, afin d'amener le mélange de composés aromatiques à huit atomes de carbone par molécule à une composition la plus proche possible de la composition correspondant à l'équilibre thermodynamique à la température considérée. Le procédé selon la présente invention s'applique à tous les catalyseurs permettant de réaliser l'isomérisation d'un mélange de composés aromatiques à 8 atomes de carbone par molécule ainsi que les catalyseurs qui permettent d'effectuer l'isomérisation désalkylante de l'éthylbenzène en benzène.

Dans le procédé selon la présente invention, le catalyseur peut être mis en oeuvre dans des gammes de température plus basses (370-420 °C) que dans les procédés classiques, à des pressions partielles d'hydrogène plus faibles (5-12 bar absolus) que dans les procédés classiques et à des PPH (poids de charge/ poids de catalyseur/ heure) plus élevés (3 à 6 h⁻¹) que dans les procédés classiques.

Les coupes traitées selon le procédé de la présente invention sont injectées avec de l'hydrogène, dans un réacteur qui contient au moins un catalyseur. Parmi les catalyseurs utilisables dans le procédé selon l'invention, on peut citer des catalyseurs comprenant au moins un métal du groupe VIII de la classification périodique des éléments (Handbook of Chemistry and Physics, 45 ème édition, 1964- 1965) comme par exemple le platine et au moins un support minéral. On utilise souvent une zéolithe comme support de catalyseur. Parmi les zéolithes qui peuvent être utilisées, on peut citer la mordénite, la zéolithe oméga, la zéolithe de type structural MFI. On peut également utiliser des tamis moléculaires non zéolithiques comme les aluminophosphates cristallins. Ces exemples ne limitent en aucun cas le choix du catalyseur utilisable.

La température de réaction est d'environ 300 à 500 °C, de préférence d'environ 350 à 450 °C et d'une manière encore plus préférée d'environ 370 à 420 °C, la pression partielle d'hydrogène est d'environ 3 à 15 bar absolus, de préférence d'environ 4 à 12 bar absolus et d'une manière encore plus préférée de 7 à 12 bar absolus, la pression totale est d'environ 4 à 20 bar absolus, de préférence de 6 à 15 bar absolus, la PPH (poids de charge/ poids de catalyseur/ heure) est d'environ 0,2 à 10 h⁻¹, de préférence d'environ 1 à 15 h⁻¹, et d'une manière encore plus préférée de 3 à 6 h⁻¹.

Dans une mise en oeuvre particulière, le procédé selon l'invention peut comprendre une étape de déshydrogénation effectuée après l'étape d'isomérisation.

Ainsi, dans un mode de réalisation particulier, la présente invention concerne un procédé d'isomérisation d'une charge contenant des aromatiques à 8 atomes de carbone comprenant au moins une étape a) d'isomérisation et au moins une étape b) de déshydrogénation.

Tout catalyseur qui permet de déshydrogéner des composés de type naphtènes en composés aromatiques peuvent être utilisés dans l'étape b) du procédé. A la sortie du réacteur de déshydrogénation, pour un nombre d'atomes de carbone par molécule donné, les composés aromatiques obtenus le sont dans les proportions de l'équilibre thermodynamique dans les conditions de température et de pression de sortie de ce réacteur.

Ainsi dans l'étape d'isomérisation de la mise en oeuvre particulière du procédé, les conditions opératoires de la zone d'isomérisation sont choisies de façon à minimiser la production de composés non désirés issus de réactions faisant intervenir des mécanismes de catalyse acide (craquage, désalkylation, dismutation...).

L'effluent obtenu à l'issue de la première étape de réaction est traité lors d'une seconde étape dans une zone réactionnelle contenant au moins un catalyseur de déshydrogénation. Les conditions opératoires de cette seconde étape peuvent être différentes ou identiques aux conditions opératoires de la première étape, de préférence les conditions opératoires de ces 2 étapes sont différentes. Les conditions opératoires de cette seconde étape sont déterminées de façon à obtenir une composition du mélange de xylènes et d'éthylbenzène la plus proche de la composition à l'équilibre thermodynamique.

Les catalyseurs de déshydrogénation des paraffines et des naphtènes sont bien connus de l'homme du métier. Les supports de ces catalyseurs sont généralement des oxydes réfractaires, le plus souvent on choisi une alumine. Ces catalyseurs de déshydrogénation comprennent au moins un métal noble du groupe VIII de la classification périodique et au moins un élément alcalin ou alcalino-terreux des groupes la et IIa de la classification périodique. De préférence, le métal noble du groupe VIII choisi est le platine, et l'élément des groupes la ou IIa de la classification périodique est choisi dans le groupe comprenant le magnésium, le potassium, le calcium.

Ces catalyseurs de déshydrogénation peuvent aussi contenir du thorium et/ou au moins un élément M des groupes IV a ou IV b de la classification périodique des éléments. Les éléments des groupes IV a ou IV b sont le plus souvent choisis dans le groupe formé par l'étain, le silicium, le titane et le zirconium. Certains catalyseurs de déshydrogénation contiennent aussi du soufre et/ou un halogène. Plus particulièrement, on peut utiliser à l'étape de déshydrogénétion du procédé selon la présente invention les catalyseurs de déshydrogénétion décrits dans les brevets US 3 998 900 et US 3 531 543.

Sans vouloir être lié à une quelconque théorie, on note que le platine présente une activité hydrogénolysante qui s'exprime au détriment de l'activité de déshydrogénation de paraffines en composés aromatiques. Cette activité hydrogénolysante peut être fortement réduite, et la sélectivité du catalyseur vis à vis de la réaction de déshydrogénation augmentée, par l'ajout de l'élément additionnel M.

Les supports inorganiques réfractaires utilisés ont souvent un caractère acide et peuvent générer des réactions secondaires indésirables telles que les réactions de craquage ou d'isomérisation. C'est pourquoi le support oxyde est généralement neutralisé par l'ajout d'au moins un élément alcalin ou alcalino-terreux.

L'étape de déshydrogénation est mise en oeuvre, en présence d'hydrogène qui peut être introduit sous forme d'hydrogène frais, sous forme d'hydrogène recyclé provenant de la sortie de la zone d'isomérisation ou sous forme d'hydrogène recyclé provenant de la sortie de la zone de déshydrogénation.

Les conditions opératoires pour l'étape de déshydrogénation sont une température d'environ 300 à 500°C, de préférence d'environ 400 à 420°C, une pression partielle absolue d'hydrogène d'environ 1 à 15 bar, de préférence d'environ 4 à 10 bar, une pression totale absolue d'environ 2 à 20 bar, de préférence d'environ 5 à 15 bar et un PPH (poids de charge/ poids de catalyseur/ heure) d'environ 0,2 à 10 h⁻¹, de préférence d'environ 3 à 6 h⁻¹.

D'autre part, on peut aussi effectuer un recyclage des composés aromatiques à 8 atomes de carbone contenus dans l'effluent de la zone de déshydrogénation après avoir extrait les composés recherchés c'est-à-dire le paraxylène et éventuellement l'orthoxylène.

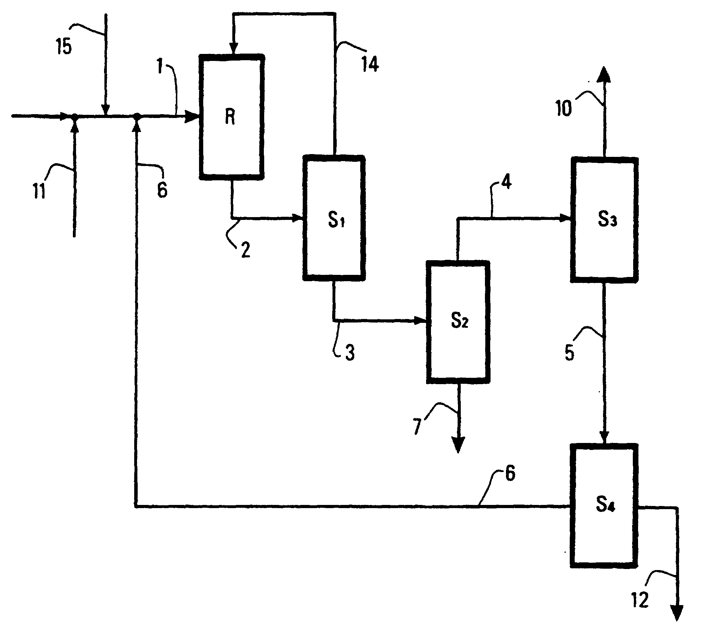

La figure 1 présente une réalisation simple du procédé selon l'invention.

Suivant cette figure, la charge fraîche, contenant le mélange de composés aromatiques à 8 atomes de carbone à isomériser est introduite dans le réacteur R par la ligne 1. Avant d'être injectée dans le réacteur d'isomérisation R, cette charge fraîche est enrichie en un mélange contenant les composés suivants : au moins une paraffine à huit atomes de carbone par molécule , du benzène, du toluène et au moins un naphtène à huit atomes de carbone par molécule. Ces ajouts sont effectués par un recyclage assuré par la ligne 6 ou par l'addition des composés frais par la ligne 11 ou encore par une combinaison de composés frais et recyclés assurée par ces deux lignes 6 et 11. Un appoint d'hydrogène est assuré par la ligne 15.

Après réaction, l'effluent est envoyé dans une zone de séparation S₁. Dans cette zone S₁, on isole l'hydrogène contenu dans l'effluent et on le recycle vers l'entrée du réacteur par la ligne 14, le reste de l'effluent est envoyé dans une zone de séparation S₂ par une ligne 3. Dans cette zone de séparation S₂, on sépare les produits de la réaction en deux fractions : une fraction légère qui contient les paraffines, les naphtènes ainsi que les composés aromatiques les plus légers comme le benzène et le toluène est envoyée par la ligne 4 dans une zone de séparation S₃; l'autre fraction comprend les composés aromatiques contenant au moins huit atomes de carbone. De cette fraction seront extraits, après des étapes successives de séparation, les produits désirés en particulier le paraxylène.

Dans une zone de séparation S₃, on sépare les hydrocarbures contenant de un à sept atomes de carbone par molécule, ils sont évacués par la ligne 10, de la phase contenant les paraffines à huit atomes de carbone par molécule, les naphtènes à huit atomes de carbone par molécule, le benzène et le toluène. Ce mélange est envoyé par la ligne 5 dans une zone de séparation S₄. Dans la zone de séparation S₄, on choisit les quantités en paraffines à huit atomes de carbone par molécule, en benzène, en toluène et en naphtènes à huit atomes de carbone par molécule que l'on veut recycler. Ce mélange est ensuite envoyé par la ligne 6 en amont du réacteur où il enrichit la charge fraîche (à cette charge fraîche a été précédemment ajoutée une partie de la fraction, issue de S₂ qui comprend les composés aromatiques contenant au moins huit atomes de carbone par molécule et dont on a extrait les produits désirés en particulier le paraxylène). Une ligne 12 est prévue pour évacuer la partie du mélange comprenant des paraffines à huit atomes de carbone par molécule, des naphtènes à huit atomes de carbone par molécule, du benzène et du toluène que l'on ne désire pas recycler.

Les exemples suivants illustrent l'invention sans en limiter la portée.

### Exemples

Le catalyseur utilisé dans les exemples suivants est un catalyseur à base d'alumine contenant de la mordénite ainsi que 0,4 % en poids de platine, ce catalyseur se présente sous forme d'extrudés.

### Exemples 1 et 2 (comparatif)

Les exemples 1 et 2 ont été réalisés dans les conditions opératoires suivantes : une température de 385 °C, une pression totale de 8 bar absolus avec un rapport molaire hydrogène sur hydrocarbures de 4/1.

L'exemple 1 a été réalisé sur une charge 1 dont la composition est consignée dans le tableau suivant. Dans l'exemple 2, une charge 2 a été traitée, cette charge est sensiblement identique à la charge 1 cependant 1% en poids de paraffines à huit atomes de carbone par molécule par rapport à la charge totale qui entre dans le réacteur a été ajouté.

| Composés | EXEMPLE 1 | | EXEMPLE 2 | |
|---|---|---|---|---|
| | Charge 1 (% poids) | Produits (% poids) | Charge 2 (% poids) | Produits (% poids) |
| Paraffines C1-C4 | - | 0,45 | - | 0,7 |
| i-Pentane | - | 0,1 | - | 0,2 |
| n-Pentane | - | 0,1 | - | 0,1 |
| Benzène | - | 0,3 | - | 0,3 |
| Toluène | 0,2 | 1,1 | 0,25 | 0,9 |
| o-Xylène | 25,3 | 18,8 | 25,1 | 19,4 |
| m-Xylène | 56,5 | 42,0 | 56,3 | 42,4 |
| p-Xylène | 1,5 | 17,7 | 1,5 | 16,8 |
| Ethylbenzène | 14,0 | 9,0 | 13,6 | 9,7 |
| Naphtènes C6 | - | 0,2 | - | 0,1 |
| Naphtènes C7 | - | 0,2 | - | 0,1 |
| Paraffines C8 | 0,1 | 0,37 | 1,1 | 1,2 |
| Naphtènes C8 | 2,0 | 7,6 | 1,9 | 6,7 |
| Composés | 0,1 | 1,9 | - | 0,6 |
| aromatiques C9+ | | | | |

Dans l'exemple 1, les pertes sous forme de paraffines à huit atomes de carbone par molécule sont de 0,28 % en poids par rapport à la quantité de composés aromatiques à huit atomes de carbone par molécule introduite dans le réacteur, dans l'exemple 2, ces pertes sont de 0,10 % en poids.

En ajoutant 1% en poids de paraffines à huit atomes de carbone par molécule par rapport à la charge totale qui entre dans le réacteur (exemple 2), on a diminué les pertes sous forme de paraffines à huit atomes de carbone par molécule de 0,18 % en poids par rapport à l'isomérisation réalisée sans ajout (exemple 1).

### Exemples 3 et 4 (comparatif)

Les exemples 3 et 4 ont été réalisés dans les conditions opératoires suivantes : une température de 390 °C, une pression totale de 9 bar absolus avec un rapport molaire hydrogène sur hydrocarbures de 4/1. L'exemple 3 a été réalisé sur une charge 3, dont la composition est consignée dans le tableau suivant. Dans l'exemple 4, une charge 4 a été traitée, cette charge est sensiblement identique à la charge 3, cependant 1,8 % en poids de naphtènes à huit atomes de carbone par molécule par rapport à la charge totale qui entre dans le réacteur et 1,2 % en poids de toluène par rapport à la charge totale qui entre dans le réacteur ont été ajoutés.

| Composés | EXEMPLE 3 | | EXEMPLE 4 | |
|---|---|---|---|---|
| | Charge 3 (% poids) | Produits (% poids) | Charge 4 (% poids) | Produits (% poids) |
| Paraffines C1-C4 | - | 0,6 | - | 0,3 |
| i-Pentane | - | 0,1 | - | 0,2 |
| n-Pentane | - | 0,1 | - | 0,1 |
| Benzène | - | 0,3 | - | 0,3 |
| Toluène | 0,2 | 1,1 | 1,4 | 1,8 |
| o-Xylène | 25,3 | 19,1 | 24,5 | 19,3 |
| m-Xylène | 56,5 | 42,0 | 54,9 | 41,7 |
| p-Xylène | 1,5 | 17,4 | 1,5 | 16,6 |
| Ethylbenzène | 14,0 | 9,5 | 13,6 | 9,9 |
| Naphtènes C6 | - | 0,1 | - | 0,1 |
| Naphtènes C7 | - | 0,2 | - | 0,3 |
| Paraffines C8 | 0,1 | 0,3 | 0,1 | 0,3 |
| Naphtènes C8 | 2,0 | 6,9 | 3,8 | 6,75 |
| Composés | 0,1 | 1,9 | 0,1 | 1,5 |
| aromatiques C9+ | | | | |

Dans l'exemple 3, les pertes sous forme de naphtènes à huit atomes de carbone par molécule sont de 5,03 % en poids par rapport à la quantité de composés aromatiques à huit atomes de carbone introduite dans le réacteur et les pertes sous forme de toluène sont de 0,92 % en poids par rapport à la quantité de composés aromatiques à huit atomes de carbone par molécule introduite dans le réacteur, dans l'exemple 4, les pertes sous forme de naphtènes à huit atomes de carbone par molécule sont de 3,12 % en poids par rapport à la quantité de composés aromatiques à huit atomes de carbone par molécule introduite dans le réacteur et les pertes sous forme de toluène sont de 0,42 % en poids par rapport à la quantité de composés aromatiques à huit atomes de carbone par molécule introduite dans le réacteur.

En ajoutant 1,8 % en poids de naphtènes à huit atomes de carbone par molécule par rapport à la charge totale qui entre dans le réacteur et 1,2 % en poids de toluène par rapport à la charge totale (exemple 4), on a diminué les pertes sous forme de naphtènes à huit atomes de carbone par molécule de 1,91 % en poids et les pertes sous formes de toluène de 0,5 % en poids par rapport à l'isomérisation réalisée sans ajouts (exemple 3).

### Exemple 5

L'exemple 5 illustre l'influence du recyclage d'une coupe dont l'intervalle de distillation est d'environ 80 à 135 °C sur les pertes en composés aromatiques à huit atomes de carbone par molécule. Ces exemples ont été réalisés dans les conditions opératoires suivantes : une température de 385°C, une pression totale de 8 Bar absolus avec un rapport molaire hydrogène sur hydrocarbures de 4/1. Dans l'exemple 5, un recyclage correspondant à 9% en poids de la coupe qui sort du réacteur R et qui contient un mélange comprenant des paraffines à huit atomes de carbone par molécule, du benzène, du toluène et des naphtènes à huit atomes de carbone par molécule obtenu après séparation à partir de l'effluent du réacteur R est ajoutée par la ligne 6 à la charge qui entre dans le réacteur. La composition de la charge 5 de l'exemple 5 est consignée dans le tableau ci-dessous.

| Composés | EXEMPLE 5 | |
|---|---|---|
| | Charge 5 (% poids) | Produits (% poids) |
| Paraffines C1-C4 | - | 0,7 |
| i-Pentane | - | 0,2 |
| n-Pentane | - | 0,1 |
| Benzène | 0,1 | 0,3 |
| Toluène | 2,0 | 2,4 |
| o-Xylène | 22,8 | 18,7 |
| m-Xylène | 52,0 | 41,3 |
| p-Xylène | 1,6 | 16,8 |
| Ethylbenzène | 13,2 | 9,4 |
| Naphtènes C6 | - | 0,2 |
| Naphtènes C7 | 0,1 | 0,5 |
| Paraffines C8 | 0,4 | 0,5 |
| Naphtènes C8 | 7,5 | 7,3 |
| Composés | 0,1 | 1,6 |
| aromatiques C9+ | | |

Dans l'exemple 1, les pertes sous forme de naphtènes à huit atomes de carbone sont de 5,75 % en poids par rapport à la quantité de composés aromatiques à huit atomes de carbone par molécule introduite dans le réacteur, les pertes sous forme de composés aromatiques et de naphtènes dont la chaîne carbonée contient un nombre d'atomes de carbone par molécule autre que huit (ce sont essentiellement des pertes sous forme de benzène, de toluène, de naphtènes à six et sept atomes de carbone et des composés aromatiques contenant au moins neuf atomes de carbone) sont de 3,49 % en poids par rapport à la quantité de composés aromatiques à huit atomes de carbone par molécule introduite dans le réacteur et le rendement en composés aromatiques à 8 atomes de carbone est de 90% en poids. Dans l'exemple 5, les pertes sous forme de naphtènes à huit atomes de carbone par molécule sont de 0,22 % en poids par rapport à la quantité de composés aromatiques à huit atomes de carbone par molécule introduite dans le réacteur, les pertes sous forme de composés aromatiques et de naphtènes dont la chaîne carbonée contient un nombre d'atomes de carbone par molécule autre que huit sont de 3,01 % en poids par rapport à la quantité de composés aromatiques à huit atomes de carbone par molécule introduite dans le réacteur.

Le rendement en composés aromatiques à huit atomes de carbone par molécule est de 96,2 % en poids.

Ainsi, en ajoutant un recyclage correspondant à 9% en poids de la coupe qui sort du réacteur R et qui contient un mélange comprenant des paraffines à huit atomes de carbone par molécule, du benzène, du toluène et des naphtènes à huit atomes de carbone par molécule obtenu après séparation à partir de l'effluent du réacteur R à la charge qui entre dans le réacteur (exemple 5), on a diminué les pertes en naphtènes à huit atomes par molécule de 5,53 % en poids et les pertes sous forme de composés aromatiques et de naphtènes dont la chaîne carbonée contient un nombre d'atomes de carbone par molécule autre que huit de 0,48% en poids par rapport à l'isomérisation réalisée sans ajouts (exemple 1). Le rendement pondéral en composés aromatiques à huit atomes de carbone par molécule a été augmenté de 6,2 %.

### Exemple 6

L'exemple 6 illustre l'influence du recyclage d'une coupe dont l'intervalle de distillation est d'environ 80 à 135 °C combiné à une addition de toluène sur les pertes en composés aromatiques à huit atomes de carbone par molécule. Ces exemples ont été réalisés dans les conditions opératoires suivantes : une température de 385 °C, une pression totale de 8 bar absolus avec un rapport molaire hydrogène sur hydrocarbures de 4/1. Dans l'exemple 6, un recyclage correspondant à 9% en poids de la coupe qui sort du réacteur R et qui contient un mélange comprenant des paraffines à huit atomes de carbone par molécule, du benzène, du toluène et des naphtènes à huit atomes de carbone par molécule obtenu après séparation à partir de l'effluent du réacteur R est ajoutée par la ligne 6 à la charge qui entre dans le réacteur. On ajoute de plus à la charge qui entre dans le réacteur 1% en poids de toluène frais. La composition de la charge 6 de l'exemple 6 est consignée dans le tableau ci-dessous :

| Composés | EXEMPLE 6 | |
|---|---|---|
| | Charge 6 (% poids) | Produits 6 (% poids) |
| Paraffines C1-C4 | - | 0,6 |
| i-Pentane | - | 0,2 |
| n-Pentane | - | 0,1 |
| Benzène | 0,1 | 0,3 |
| Toluène | 2,9 | 3,1 |
| o-Xylène | 22,7 | 18,6 |
| m-Xylène | 51,6 | 40,9 |
| p-Xylène | 1,6 | 16,8 |
| Ethylbenzène | 13,0 | 9,2 |
| Naphtènes C6 | - | 0,2 |
| Naphtènes C7 | 0,1 | 0,6 |
| Paraffines C8 | 0,4 | 0,5 |
| Naphtènes C8 | 7,4 | 7,2 |
| Composés | - | 1,6 |
| aromatiques C9+ | | |

Dans l'exemple 6, les pertes sous forme de naphtènes à huit atomes de carbone par molécule sont de 0,22 % en poids par rapport à la quantité de composés aromatiques à huit atomes de carbone par molécule introduite dans le réacteur, les pertes sous forme de composés aromatiques et de naphtènes dont la chaîne carbonée contient un nombre d'atomes de carbone par molécule autre que huit sont de 3,03 % en poids par rapport à la quantité de composés aromatiques à huit atomes de carbone par molécule introduite dans le réacteur et le rendement en composés aromatiques à 8 atomes de carbone par molécule est de 96,2% en poids. Ainsi, en ajoutant un recyclage correspondant à 9% en poids de la coupe qui sort du réacteur R et qui contient un mélange comprenant des paraffines à huit atomes de carbone par molécule, du benzène, du toluène et des naphtènes à huit atomes de carbone par molécule obtenu après séparation à partir de l'effluent du réacteur R ainsi que 1% en poids de toluène frais à la charge qui entre dans le réacteur, on a diminué les pertes en naphtènes à huit atomes de carbone par molécule de 5,52 % en poids et les pertes sous forme de composés aromatiques et de naphtènes dont la chaîne carbonée contient un nombre d'atomes de carbone par molécule autre que huit de 0,56% en poids par rapport à l'isomérisation réalisée sans ajouts (exemple 1). Le rendement en composés aromatiques à huit atomes de carbone par molécule a été augmenté de 6,2%.

Les résultats des exemples sont récapitulés dans le tableau suivant :

| | Comparatifs | | | | | |
|---|---|---|---|---|---|---|
| Pertes (% en poids) | Exemple 1 | Exemple 2 | Exemple 3 | Exemple 4 | Exemple 5 | Exemple 6 |
| en paraffines à 8 atomes de carbone | 0,28 | 0,1 | 0,2 | 0,21 | 0,11 | 0,11 |
| en naphtènes à 8 atomes de carbone | 5,75 | 5 | 5,03 | 3,12 | 0,22 | 0,22 |
| en toluène | 0,92 | 0,67 | 0,92 | 0,42 | 0,45 | 0,22 |
| rendement en aromatiques à 8 atomes de carbone (% en poids) | 90 | 91,5 | 90,4 | 92,6 | 96,2 | 96,2 |

Les exemples 1 et 3 ont été effectués sans ajouts à la charge, les exemples 2 et 4 ont été effectués avec des ajouts spécifiques comme il a déjà été effectué dans l'art antérieur, les exemples 5 et 6 ont été effectués avec des ajouts de composés et notamment avec un recyclage d'une fraction de l'effluent du réacteur, ces exemples 5 et 6 sont conformes à l'invention. On constate que le procédé selon l'invention permet de diminuer les pertes notamment en paraffines à 8 atomes de carbone par molécule, en naphtènes à 8 atomes de carbone par molécule et en toluène, ce procédé permet aussi d'augmenter le rendement en aromatiques à 8 atomes de carbone par molécule.

## Revendications

1. Procédé d'isomérisation des composés aromatiques à huit atomes de carbone par molécule **caractérisé en ce qu'**en entrée de réacteur on ajoute à la charge à traiter de l'hydrogène ainsi qu'un mélange constitué de composés dont le point d'ébullition est compris entre 80 et 135°C comprenant au moins une paraffine à huit atomes de carbone par molécule, au moins un naphtène à huit atomes de carbone par molécule, du benzène et du toluène et dépourvu d'hydrocarbures aromatiques à au moins 8 atomes de carbone et d'hydrocarbures ayant 1 à 7 atomes de carbone, ledit mélange étant obtenu par recyclage de la fraction contenant lesdits composés isolée à partir de l'effluent de sortie du réacteur d'isomérisation et le catalyseur utilisé étant un catalyseur comprenant au moins un métal du groupe VIII de la classification périodique des éléments et au moins un support.

2. Procédé d'isomérisation des composés aromatiques à huit atomes de carbone par molécule selon la revendication 1 **caractérisé en ce que** ledit mélange ajouté à la charge contenant au moins une paraffine à huit atomes de carbone par molécule, du benzène, du toluène et au moins un naphtène à huit atomes de carbone par molécule est obtenu par un recyclage de la fraction contenant ces composés isolée à partir de l'effluent de sortie du réacteur d'isomérisation et par des ajouts de produits frais.

3. Procédé d'isomérisation des composés aromatiques à huit atomes de carbone par molécule selon l'une des revendications 1 à 2 **caractérisé en ce que** ledit mélange d'hydrocarbures ajouté à la charge fraîche est tel que le pourcentage en poids de composés totaux ajoutés est d'environ 0,8 à 20 %.

4. Procédé d'isomérisation des composés aromatiques à huit atomes de carbone par molécule selon l'une des revendications 1 à 3 **caractérisé en ce que** le mélange d'hydrocarbures ajouté à la charge fraîche est tel que le pourcentage en poids de paraffines à huit atomes de carbone par molécule ajoutées par rapport à la charge totale qui entre dans le réacteur est d'environ 0,1 à 10, le pourcentage en poids de naphtènes à huit atomes de carbone par molécule ajoutés par rapport à la charge totale qui entre dans le réacteur est d'environ 0,5 à 15, le pourcentage en poids de toluène ajouté par rapport à la charge totale qui entre dans le réacteur est d'environ 0,1 à 10, le pourcentage en poids de benzène par rapport à la charge totale qui entre dans le réacteur est d'environ 0,1 à 10.

5. Procédé d'isomérisation des composés aromatiques à huit atomes de carbone par molécule selon l'une des revendications 1 à 4 **caractérisé en ce que** la température de réaction est d'environ 300 à 500°C, la pression partielle d'hydrogène est d'environ 3 à 15 bars absolus, la pression totale est d'environ 4 à 20 bars absolus, la PPH (poids de charge/poids de catalyseur/heure) est d'environ 0,2 à 10 h⁻¹.

6. Procédé d'isomérisation des composés aromatiques à huit atomes de carbone par molécule selon l'une des revendications 1 à 5 **caractérisé en ce que** le support du catalyseur comprend une zéolithe.

7. Procédé d'isomérisation des composés aromatiques à huit atomes de carbone par molécule selon l'une des revendications 1 à 6 **caractérisé en ce que** le support du catalyseur est un tamis moléculaire non zéolitique.

8. Procédé d'isomérisation des composés aromatiques à huit atomes de carbone par molécule selon l'une des revendications 1 à 7 **caractérisé en ce qu'**il comprend une étape de déshydrogénation effectuée après l'étape d'isomérisation.

## Patentansprüche

1. Isomerisierungsverfahren der aromatischen Verbindungen mit acht Kohlenstoffatomen pro Molekül, **dadurch gekennzeichnet, dass** man am Reaktoreingang zu der zu behandelnden Charge Wasserstoff sowie ein Gemisch zugibt, das aus Verbindungen besteht, deren Siedepunkt zwischen 80 und 135°C liegt, und welches wenigstens ein Paraffin mit acht Kohlenstoffatomen pro Molekül, wenigstens ein Naphten mit acht Kohlenstoffatomen pro Molekül, Benzol und Toluol umfasst und frei von aromatischen Kohlenwasserstoffen mit wenigstens acht Kohlenstoffatomen und von Kohlenwasserstoffen mit 1 bis 7 Kohlenstoffatomen ist, wobei dieses Gemisch durch Rezyklierung der diese Verbindungen enthaltenden Fraktion, isoliert ausgehend vom Ausgangsabstrom des Reaktors zur Isomerisierung, erhalten wird, und dass der verwendete Katalysator ein Katalysator ist, welcher wenigstens ein Metall der Gruppe VIII des Periodensystems der Elemente und wenigstens einen Träger umfasst.

2. Isomerisierungsverfahren der aromatischen Verbindungen mit acht Kohlenstoffatomen pro Molekül nach Anspruch 1, **dadurch gekennzeichnet, dass** das Gemisch, das zu der Charge gegeben wird, die wenigstens ein Paraffin mit acht Kohlenstoffatomen pro Molekül, Benzol, Toluol enthält, und wenigstens ein Naphten mit acht Kohlenstoffatomen pro Molekül durch ein Rezyklieren der ausgehend vom Ausgangsabstrom des Isomerisierungsreaktors und durch Zugaben frischer Produkte isolierten Fraktion erhalten wird, welche diese Verbindungen enthält.

3. Isomerisierungsverfahren der aromatischen Verbindungen mit acht Kohlenstoffatomen pro Molekül nach einem der Ansprüche 1 bis 2, **dadurch gekennzeichnet, dass** das zu der frischen Charge zugegebene Kohlenwasserstoffgemisch derart ist, dass der Gewichtsprozentanteil von insgesamt zugegebenen Verbindungen etwa 0,8 bis 20% beträgt.

4. Isomerisierungsverfahren der aromatischen Verbindungen mit acht Kohlenstoffatomen pro Molekül nach einem der Ansprüche 1 bis 3, **dadurch gekennzeichnet, dass** das zu der frischen Charge zugegebene Kohlenwasserstoffgemisch derart ist, dass der Gewichtsprozentanteil von Paraffin mit acht Kohlenstoffatomen pro Molekül, welche zugegeben werden im Verhältnis zur Gesamtcharge, die in den Reaktor eintritt, etwa 0,1 bis 10 ist, dass der Gewichtsanteil von zugegebenen Naphtenen mit acht Kohlenstoffatomen pro Molekül, im Verhältnis zur Gesamtcharge, die in den Reaktor eintritt, etwa 0,5 bis 15 ist, der Gewichtsprozentanteil von Toluol, das zugegeben wird, im Verhältnis zur Gesamtcharge, die in den Reaktor eintritt, etwa 0,1 bis 10 ist, der Gewichtsprozentanteil von Benzol im Verhältnis zur Gesamtcharge, die in den Reaktor eintritt, etwa 0,1 bis 10 ist.

5. Isomerisierungsverfahren der aromatischen Verbindungen mit acht Kohlenstoffatomen pro Molekül nach einem der Ansprüche 1 bis 4, **dadurch gekennzeichnet, dass** die Reaktionstemperatur etwa 300 bis 500°C ist, der Wasserstoffpartialdruck etwa 3 bis 15 bar absolut ist, der Gesamtdruck etwa 4 bis 20 bar absolut ist, die PPH (Chargengewicht/Katalysatorgewicht/Stunde) etwa 0,2 bis 10 h⁻¹ ist.

6. Isomerisierungsverfahren der aromatischen Verbindungen mit acht Kohlenstoffatomen pro Molekül nach einem der Ansprüche 1 bis 5, **dadurch gekennzeichnet, dass** der Träger des Katalysators einen Zeolith umfasst.

7. Isomerisierungsverfahren der aromatischen Verbindungen mit acht Kohlenstoffatomen pro Molekül nach einem der Ansprüche 1 bis 6, **dadurch gekennzeichnet, dass** der Träger des Katalysators ein nicht zeolithisches Molekularsieb ist.

8. Isomerisierungsverfahren von aromatischen Verbindungen mit acht Kohlenstoffatomen pro Molekül nach einem der Ansprüche 1 bis 7, **dadurch gekennzeichnet, dass** es eine Dehydrierungsstufe umfasst, die nach der Isomerisierungsstufe durchgeführt wird.

## Claims

1. A process for isomerising aromatic compounds containing eight carbon atoms per molecule, **characterized in that** hydrogen is added to the feed to be treated at the reactor inlet, also a mixture containing compounds with a boiling point of about 80°C to 135°C comprising at least one paraffin containing eight carbon atoms per molecule, at least one naphthene containing eight carbon atoms per molecule, benzene and toluene, and devoid of aromatic hydrocarbons containing at least eight carbon atoms per molecule and hydrocarbons containing 1 to 7 carbon atoms, said mixture being obtained by recycling a fraction containing said compounds isolated from the effluent leaving the isomerisation reactor, the catalyst used being a catalyst comprising at least one metal from group VIII of the periodic table and at least one support.

2. A process for isomerising aromatic compounds containing eight carbon atoms per molecule according to claim 1, **characterized in that** the mixture added to the feed containing at least one paraffin containing eight carbon atoms per molecule, benzene, toluene and at least one naphthene containing eight carbon atoms per molecule is obtained by recycling the fraction containing said compounds isolated from the effluent leaving the isomerisation reactor and by adding fresh products.

3. A process for isomerising aromatic compounds containing eight carbon atoms per ' molecule according to any one of claims 1 to 2, **characterized in that** the mixture of hydrocarbons added to the fresh feed is such that the total percentage by weight of the added compounds is about 0.8% to 20%.

4. A process for isomerising aromatic compounds containing eight carbon atoms per molecule according to any one of claims 1 to 3, **characterized in that** the mixture of hydrocarbons added to the fresh feed is such that the percentage by weight of paraffins containing eight carbon atoms per molecule added with respect to the total feed entering the reactor is about 0.1% to 10%, the percentage by weight of naphthenes containing eight carbon atoms per molecule added with respect to the total feed entering the reactor is about 0.5% to 15%, the percentage by weight of toluene added with respect to the total feed entering the reactor is about 0.1% to 10%, and the percentage by weight of benzene with respect to the total feed entering the reactor is about 0.1% to 10%.

5. A process for isomerising aromatic compounds containing eight carbon atoms per molecule according to any one of claims 1 to 4, **characterized in that** the reaction temperature is about 300°C to 500°C, the partial pressure of hydrogen is about 3 to 15 bars absolute, the total pressure is about 4 to 20 bars absolute, and the HSV (weight of feed/weight of catalyst/hour) is about 0.2 to 10 h⁻¹.

6. A process for isomerising aromatic compounds containing eight carbon atoms per. molecule according to any one of claims 1 to 5, **characterized in that** the catalyst support comprises a zeolite.

7. A process for isomerising aromatic compounds containing eight carbon atoms per molecule according to any one of claims 1 to 6, **characterized in that** the catalyst support is a non zeolitic molecular sieve.

8. A process for isomerising aromatic compounds containing eight carbon atoms per molecule according to any one of claims 1 to 7 **characterized in that** it comprises a dehydrogenation step carried out after the isomerization step.
